**Europäisches Patentamt**

**European Patent Office**

**Office européen des brevets**

(19)

(11) Veröffentlichungsnummer : **0 304 730 B1**

# EUROPÄISCHE PATENTSCHRIFT

(12)

(45) Veröffentlichungstag der Patentschrift :
26.06.91 Patentblatt 91/26

(51) Int. Cl.$^5$ : **A61K 31/46, A61K 31/21**

(21) Anmeldenummer : **88113031.4**

(22) Anmeldetag : **11.08.88**

(54) **N-Alkylscopolaminiumsalz und Nitroglycerin enthaltendes Arzneimittel, Verfahren zu dessen Herstellung und dessen Verwendung.**

(30) Priorität : **27.08.87 DE 3728563**

(43) Veröffentlichungstag der Anmeldung :
**01.03.89 Patentblatt 89/09**

(45) Bekanntmachung des Hinweises auf die
Patenterteilung :
**26.06.91 Patentblatt 91/26**

(84) Benannte Vertragsstaaten :
**AT BE CH DE ES FR GB GR IT LI LU NL SE**

(56) Entgegenhaltungen :
**EP-A- 0 013 606**
**DE-A- 2 920 500**
**GB-A- 1 427 881**
**P.H.LIST, L. HÖRHAMMER "Hagers Handbuch
der Pharmazeuischen Praxis" 4. Neuausgabe,
Band II, 1969 SPRINGER-VERLAG Berlin, Heidelberg, New York Seiten 484-485**

(73) Patentinhaber : **G. Pohl-Boskamp GmbH & Co.
Chemisch-pharmazeutische Fabrik
Kieler Strasse 11
W-2214 Hohenlockstedt (DE)**

(72) Erfinder : **Dunzendorfer, Udo, Dr.med.
Scheffelstrasse 1-16
W-6000 Frankfurt/Main (DE)**
Erfinder : **Cholcha, Walter, Dr.
Kaltenweide 70e
W-2200 Elmshorn (DE)**

(74) Vertreter : **UEXKÜLL & STOLBERG
Patentanwälte
Beselerstrasse 4
W-2000 Hamburg 52 (DE)**

**Beschreibung**

Die Erfindung betrifft ein Arzneimittel, das N-Alkylscopolaminiumsalz mit 1 bis 10 Kohlenstoffatomen im Alkylrest, vorzugsweise N-Butylscopolaminiumbromid, als Wirkstoff sowie gegebenenfalls übliche Hilfs- und Trägerstoffe enthält, ein Verfahren zu dessen Herstellung und dessen Verwendung.

Zur Behandlung von Kolikschmerzen, vornehmlich im Bereich der abführenden Harnwege, der Gallenblase und der Gallenwege wurden bisher insbesondere Kombinationspräparate auf Basis von Metamizol erfolgreich angewandt. Die Wirkstoffkombination in derartigen Präparaten bestand beispielsweise aus N-Butylscopolaminiumbromid und Metamizol. Es hat sich allerdings herausgestellt, daß Metamizol enthaltende Kombinationspräparate bedenkliche Nebenwirkungen zeigen, so daß beispielsweise in der Bundesrepublik Deutschland die Zulassung dieser Präparate widerrufen worden ist. Es besteht deshalb ein erheblicher Bedarf einen Ersatz für die nicht mehr zulässigen Kombinationspräparate auf Basis von Metamizol zu finden.

Dementsprechend liegt der Erfindung die Aufgabe zugrunde, ein Arzneimittel zu liefern, daß sich ebenso gut für die Therapie der oben genannten Kolikschmerzen eignet, wie die nicht mehr zulässigen Kombinationspräparate auf Basis von Metamizol.

Zur Lösung dieser Aufgabe wird ein Arzneimittel der eingangs genannten Art vorgeschlagen, das dadurch gekennzeichnet ist, daß es als weiteren Wirkstoff Nitroglycerin enthält.

Gegenstand der Erfindung ist ferner ein Verfahren zur Herstellung des erfindungsgemäßen Arzneimittels, das dadurch gekennzeichnet ist, daß man

1a) eine wäßrige Lösung von N-Alkylscopolaminiumsalz, vorzugsweise N-Butylscopolaminiumbromid, und Nitroglycerin herstellt, indem man etwa 90% der insgesamt vorgesehenen Wassermenge vorlegt, auf einen pH-Wert von 3,0-6,5 ansäuert, auf 90-95°C erhitzt, Nitroglycerin als solches, als Lösung oder aufgebracht auf ein inertes Trägermaterial unter Rühren vorsichtig einträgt, nach vollständiger Auflösung des Nitroglycerins N-Alkylscopolaminiumsalz zu der Lösung gibt und nach erfolgter Auflösung des N-Alkylscopolaminiumsalzes auf Raumtemperatur abkühlt und mit Wasser auf das gewünschte Endvolumen auffüllt, oder

1b) eine organische Lösung oder Suspension von N-Alkylscopolaminiumsalz, vorzugsweise N-Butylscopolaminiumbromid, herstellt, indem man das N-Alkylscopolaminiumsalz vorlegt und mit der 1- bis 200-fachen Menge eines organischen Lösungsmittels unter leichtem Erwärmen im Wasserbad versetzt und zu der resultierenden, auf Raumtemperatur abgekühlten Lösung eine Lösung von Nitroglycerin in Alkohol oder mittelkettigen Triglyceriden gibt, und gegebenenfalls

2) die erhaltene auf Raumtemperatur abgekühlte Lösung von N-Alkylscopolaminiumsalz und Nitroglycerin in an sich bekannter Weise zu der gewünschten Darreichungsform des Arzneimittels weiterverarbeitet.

Schließlich ist Gegenstand der Erfindung die Verwendung von N-Alkylscopolaminiumsalz zusammen mit Nitroglycerin zur Herstellung eines Arzneimittels (Spasmolytikum) zur Behandlung von Kolikschmerzen, vornehmlich im Bereich der abführenden Harnwege, der Gallenblase und der Gallenwege.

Der Alkylrest in den erfindungsgemäß verwendeten N-Alkylscopolaminiumsalzen kann 1 bis 10 Kohlenstoffatome aufweisen. Bevorzugt sind Alkylreste mit 2 bis 6 Kohlenstoffatomen und insbesondere mit 4 Kohlenstoffatomen. Sowohl N-Alkylscopolaminiumsalze, insbesondere N-Butylscopolaminiumbromid, als auch Nitroglycerin sind im Handel erhältliche Substanzen und/oder in der Literatur beschrieben (vgl. z.B. Martin Negwer "Organisch-Chemische Arzneimittel und ihre Synonyma", 5. Auflage, Band II, 1978, Nr. 4751 auf S. 875 und Band I, Nr. 91 auf S. 15, Arzneimittel Forschung 18, S. 1132 ff., 1968 usw.), so daß sich ein Eingehen auf deren Herstellung und Eigenschaften erübrigt.

Die Herstellung der im erfindungsgemäßen Arzneimittel enthaltenen Wirkstoffkombination erfolgt vorzugsweise so, daß man zunächst eine wässrige Lösung der Wirkstoffkombination herstellt. Hierzu legt man etwa 90% der benötigten Wassermenge vor und stellt den pH-Wert, vorzugsweise mit verdünnter Salzsäure, auf 3,0-6,5 ein. Dann wird auf 90-95°C erhitzt. Die benötigte Menge Nitroglycerin wird unter Rühren vorsichtig eingetragen. Dabei kann das Nitroglycerin als solches, als Lösung oder auf einem inerten Trägermaterial, vorzugsweise als Nitroglycerin-Zuckerverreibung, eingesetzt werden (vgl. hierzu z.B. Europäische Patentanmeldung 0 108 248 und die darin genannten Druckschriften). Nach vollständiger Auflösung des Nitroglycerins wird die vorgesehene Menge N-Alkylscopolaminiumsalz, vorzugsweise N-Butylscopolaminiumbromid, hinzugegeben. Sobald sich letzteres gelöst hat, wird auf Raumtemperatur abgekühlt. Danach wird der Ansatz auf das gewünschte Endvolumen aufgefüllt.

Alternativ kann man eine organische Lösung oder Suspension von N-Alkylscopolaminiumsalz herstellen, indem man das N-Alkylscopolaminiumsalz, vorzugsweise N-Butylscopolaminiumbromid, vorlegt und mit der 1- bis 200-fachen Menge eines organischen Lösungsmittels, vorzugsweise Ethanol, Polyethylenglykol, Propylenglykol oder Glycerin, unter leichtem Erwärmen im Wasserbad versetzt. Zu der erhaltenen Lösung wird dann nach Abkühlung auf Raumtemperatur eine Lösung von Nitroglycerin in Alkohol oder mittelkettigen Triglyceriden gegeben. Der Begriff "mit-

telkettige Triglyceride" bezeichnet Triglyceride, in denen die Fettsäuren 4 bis 12 Kohlenstoffatome aufweisen.

Die so erhaltene wäßrige Lösung der erfindungsgemäßen Wirkstoffkombination kann bereits als solche oder nach Weiterverarbeitung in an sich bekannter Weise eingesetzt werden. Die an sich bekannte Weiterverarbeitung kann darin bestehen, daß man die Lösung, wenn sie z.B. zur parenteralen Applikation vorgesehen ist, steril filtriert, abfüllt und sterilisiert. Eine andere Weiterverarbeitungsmöglichkeit der wäßrigen oder organischen Lösung besteht darin, daß man die Lösung mit geeigneten Hilfsstoffen versetzt und in eine Sprayflasche oder Spraydose abfüllt, so daß das Arzneimittel als Pumpspray verwendet werden kann. Wenn man zusätzlich ein Treibmittel verwendet, kann das Arzneimittel auch als Druckaerosol verwendet werden. Weiterhin kann die Lösung der erfindungsgemäßen Wirkstoffkombination unter Zusatz geeigneter Hilfsstoffe zu einem Arzneimittel in Form einer Weichgelatinekapsel oder einer Rektalkapsel verarbeitet werden.

Das Gewichtsverhältnis von N-Alkylscopolaminiumsalz zu Nitroglycerin in dem erfindungsgemäßen Arzneimittel beträgt vorzugsweise 50 : 1 bis 2 : 1 und insbesondere 10 : 1 bis 7 : 1. Bei Verwendung von N-Butylscopolaminiumsalz sind besonders gute Ergebnisse sind bei einem Gewichtsverhältnis von 8,3 : 1 erzielt worden. Eine Dosiseinheit des erfindungsgemäßen Arzneimittels enthält vorzugsweise 10-30 mg, insbesondere 18-22 mg N-Alkylscopolaminiumsalz und vorzugsweise 0,4-4 mg, insbesondere 1-3 mg Nitroglycerin. Besonders gute Ergebnisse sind mit einer Dosiseinheit erzielt worden, die 20 mg N-Butylscopolaminiumbromid und 2,4 mg Nitroglycerin enthielt.

Wie bereits oben erwähnt, kann das erfindungsgemäße Arzneimittel in Form einer wässrigen Lösung parenteral und insbesondere intravenös appliziert werden. Als weitere Darreichungsformen kommen in Frage Sprays, Kapseln, Rektalkapseln, Salben, Pflaster, Tabletten, Dragees und Pillen.

Es ist für den Fachmann überraschend, daß die Kombination aus N-Alkylscopolaminiumsalz und Nitroglycerin in ihrer spasmolytischen Wirksamkeit den bekannten Kombinationspräparaten auf Basis von Metamizol entspricht und weniger unerwünschte Wirkungen als die Metamizolkombinationspräperate zeigt. Weder N-Alkylscopolaminiumsalz noch Nitroglycerin allein haben eine derartig stark ausgeprägte spasmolytische Wirkung. Hinzu kommt, daß sie gegenüber Analgetika in Form von Opioiden, die auch als Ersatz für die bisher verwendeten Kombinationspräparate auf Basis von Metamizol dienen können, den Vorteil bieten, eine kurzfristige klinische Nachuntersuchung zu ermöglichen, während Opioide das klinische Bild verschleiern und kurzfristige Kontrolluntersuchungen bzw. deren Zuverlässigkeit

beeinträchtigen.

Beispiel

Dosiseinheiten, die 20 mg N-Butylscopolaminiumbromid und 2,4 mg Nitroglycerin enthielten, wurden Harnleiterkolikpatienten, die nach einem starken Spasmolytikum verlangten und bereits verschiedene Medikamente als wirkungslos eingestuft hatten, intravenös appliziert. Nach 20 bis 35 min konnte bei allen zehn Patienten eine deutliche Spasmolyse und Schmerzlinderung erreicht werden. Klinische Komplikationen wie Steinperforation oder Urosepsis traten dabeinicht auf.

**Ansprüche**

**Patentansprüche für die Vertragsstaaten BE, DE, FR, IT, LU, NL, AT, SE, CH (LI), GB**

1. Arzneimittel, das N-Alkylscopolaminiumsalz mit 1 bis 10 Kohlenstoffatomen im Alkylrest, vorzugsweise N-Butylscopolaminiumbromid, als Wirkstoff sowie gegebenenfalls übliche Hilfs- und Trägerstoffe enthält, **dadurch gekennzeichnet,** daß es als weiteren Wirkstoff Nirtroglycerin enthält.

2. Arzneimittel nach Anspruch 1, **dadurch gekennzeichnet,** daß es N-Alkylscopolaminiumsalz und Nitroglycerin in einem Gewichtsverhältnis von 50 : 1 bis 2 : 1 und vorzugsweise 10 : 1 bis 7 : 1 enthält.

3. Arzneimittel nach Anspruch 1 oder 2, **dadurch gekennzeichnet,** daß eine Dosiseinheit 10-30 mg und vorzugsweise 18-22 mg N-Alkylscopolaminiumsalz und 0,4-4 mg und vorzugsweise 1-3 mg Nitroglycerin enthält.

4. Verfahren zur Herstellung eines Arzneimittels gemäß einem der Ansprüche 1 bis 3, **dadurch gekennzeichnet,** daß man

1a) eine wäßrige Lösung von N-Alkylscopolaminiumsalz mit 1 bis 10 Kohlenstoffatomen im Alkylrest, vorzugsweise N-Butylscopolaminiumbromid, und Nitroglycerin herstellt, indem man etwa 90% der insgesamt vorgesehenen Wassermenge vorlegt, auf einen pH-Wert von 3,0-6,5 ansäuert, auf 90-95°C erhitzt, Nitroglycerin als solches, als Lösung oder aufgebracht auf ein inertes Trägermaterial unter Rühren vorsichtig einträgt, nach vollständiger Auflösung des Nitroglycerins N-Alkylscopolaminiumsalz zu der Lösung gibt und nach erfolgter Auflösung des N-Alkylscopolaminiumsalzes auf Raumtemperatur abkühlt und mit Wasser auf das gewünschte Endvolumen auffüllt, oder

1b) eine organische Lösung von N-Alkylscopolaminiumsalz, vorzugsweise N-Butylscopolaminiumbromid, herstellt, indem man das N-Alkylscopolaminiumsalz vorlegt und mit der 1- bis 200-fachen Menge eines organischen Lösungsmittels unter leichtem Erwärmen im Wasserbad versetzt und zu der resultierenden, auf Raumtemperatur abgekühlten Lösung eine Lösung von Nitroglycerin in Alkohol oder mittelkettigen Triglyceriden gibt, und gegebenenfalls 2) die erhaltene auf Raumtemperatur abgekühlte Lösung von N-Alkylscopolaminiumsalz und Nitroglycerin in an sich bekannter Weise zu der gewünschten Darreichungsform des Arzneimittels weiterverarbeitet.

5. Verfahren nach Anspruch 4, **dadurch gekennzeichnet**, daß man in Stufe 1a) anstelle von Wasser eine isotonische Lösung als Lösungsmittel verwendet.

6. Verfahren nach Anspruch 4, **dadurch gekennzeichnet**, daß man in Stufe 1b) als organisches Lösungsmittel Ethanol, Polyethylenglykol, Propylenglykol oder Glycerin verwendet.

7. Verfahren nach einem der Ansprüche 4 bis 6, **dadurch gekennzeichnet**, daß man die Weiterverarbeitung in Stufe 2) durchführt, indem man

a) die Lösung steril filtriert, abfüllt und sterilisiert,
b) die Lösung mit geeigneten Hilfsstoffen versetzt, sie in eine Sprayflasche oder Spraydose abfüllt und gegebenenfalls mit Treibmittel versieht oder
c) die Lösung mit geeigneten Hilfsstoffen versetzt und zu einem Arzneimittel in Form von Weichgelatinekapseln, Rektalkapseln, Salben, Pflastern, Dragees, Tabletten oder Pillen verarbeitet.

8. Verwendung von N-Alkylscopolaminiumsalz mit 1 bis 10 Kohlenstoffatomen im Alkylrest, vorzugsweise N-Butylscopolaminiumbromid, zusammen mit Nitroglycerin zur Herstellung eines Arzneimittels gemäß einem der Ansprüche 1 bis 3.

**Patentansprüche für die Vertragsstaaten ES und GR**

1. Verfahren zur Herstellung eines Arzneimittels, das als Wirkstoff N-Alkylscopolaminiumsalz mit 1 bis 10 Kohlenstoffatomen im Alkylrest, vorzugsweise N-Butylscopolyaminiumbromid, und als weiteren Wirkstoff Nitroglycerin sowie gegebenenfalls übliche Hilfs- und Trägerstoffe enthält, dadurch gekennzeichnet, daß man

1a) eine wäßrige Lösung von N-Alkylscopolaminiumsalz, vorzugsweise N-Butylscopolaminiumbromid, und Nitroglycerin herstellt, indem man etwa 90% der insgesamt vorgesehenen Wassermenge vorlegt, auf einen pH-Wert von 3,0-6,5 ansäuert, auf 90-95°C erhitzt, Nitroglycerin als solches, als Lösung oder aufgebracht auf ein inertes Trägermaterial unter Rühren vorsichtig einträgt, nach vollständiger Auflösung des Nitroglycerins N-Alkylscopolaminiumsalz zu der Lösung gibt und nach erfolgter Auflösung des N-Alkylscopolaminiumsalzes auf Raumtemperatur abkühlt und mit Wasser auf das gewünschte Endvolumen auffüllt, oder

1b) eine organische Lösung von N-Alkylscopolaminiumsalz, vorzugsweise N-Butylscopolaminiumbromid, herstellt, indem man das N-Alkylscopolaminiumsalz vorlegt und mit der 1- bis 200-fachen Menge eines organischen Lösungsmittels unter leichtem Erwärmen im Wasserbad versetzt und zu der resultierenden, auf Raumtemperatur abgekühlten Lösung eine Lösung von Nitroglycerin in Alkohol oder mittelkettigen Triglyceriden gibt, und gegebenenfalls 2) die erhaltene auf Raumtemperatur abgekühlte Lösung von N-Alkylscopolaminiumsalz und Nitroglycerin in an sich bekannter Weise zu der gewünschten Darreichungsform des Arzneimittels weiterverarbeitet.

2. Verfahren nach Anspruch 1, dadurch gekennzeichnet, daß man in Stufe 1a) anstelle von Wasser eine isotonische Lösung als Lösungsmittel verwendet.

3. Verfahren nach Anspruch 1, dadurch gekennzeichnet, daß man in Stufe 1b) als organisches Lösungsmittel Ethanol, Polyethylenglykol, Propylenglykol oder Glycerin verwendet.

4. Verfahren nach einem der Ansprüche 1 bis 3, dadurch gekennzeichnet, daß man die Weiterverarbeitung in Stufe 2) durchführt, indem man

a) die Lösung steril filtriert, abfüllt und sterilisiert,
b) die Lösung mit geeigneten Hilfsstoffen versetzt, sie in eine Sprayflasche oder Spraydose abfüllt und gegebenenfalls mit Treibmittel versieht oder
c) die Lösung mit geeigneten Hilfsstoffen versetzt und zu einem Arzneimittel in Form von Weichgelatinekapseln, Rektalkapseln, Salben, Pflastern, Dragees, Tabletten oder Pillen verarbeitet.

5. Verwendung von N-Alkylscopolaminiumsalz mit 1 bis 10 Kohlenstoffatomen im Alkylrest, vorzugsweise N-Butylscopolaminiumbromid, zusammen mit Nitroglycerin zur Herstellung eines Arzneimittels zur Behandlung von Kolikschmerzen im Bereich der abführenden Harnwege, der Gallenblase und der Gallenwege.

**Claims**

**Claims for the Contracting States BE, DE, FR, IT, LU, NL, AT, SE, CH (LI), GB**

1. Medicament which contains as active ingredient N-alkylscopolaminium salt with 1 to 10 carbon atoms in the alkyl residue, preferably N-butylscopolaminium bromide, as well as, optionally, conventional adjuvants and carriers, characterised in

7      EP 0 304 730 B1      8

that it contains, as a further active ingredient, nitroglycerin.

2. Medicament according to claim 1, characterised in that it contains N-alkylscopolaminium salt and nitroglycerin in a proportion by weight of 50 : 1 to 2 : 1 and preferably 10 : 1 to 7 : 1.

3. Medicament according to claim 1 or 2, characterised in that one dose unit contains 10-30 mg and preferably 18-22 mg of N-alkylscopolaminium salt and 0.4-4 mg and preferably 1-3 mg of nitroglycerin.

4. Process for preparing a medicament according to one of claims 1 to 3, characterised in that

1a) an aqueous solution of N-alkylscopolaminium salt with 1 to 10 carbon atoms in the alkyl residue, preferably N-butylscopolaminium bromide, and nitroglycerin is prepared by introducing first approximately 90% of the total intended amount of water, acidifying it to a pH value of 3.0 to 6.5, heating it to 90-95°C, carefully introducing, whilst stirring, nitroglycerin as such, as a solution or deposited on an inert carrier material, adding N-alkylscopolaminium salt to the solution after the nitroglycerin has completely dissolved and, when the N-alkylscopolaminium salt has been dissolved, cooling the solution to room temperature and making it up with water to the desired final volume, or

1b) preparing an organic solution of N-alkylscopolaminium salt, preferably N-butylscopolaminium bromide, by introducing first the N-alkylscopolaminium salt and reacting it with 1 to 200 times the amount of an organic solvent with gentle warming in a water bath and adding to the resulting solution cooled to room temperature, a solution of nitroglycerin in alcohol or medium-chain-length triglycerides, and, optionally 2) processing in a manner known per se the obtained solution of N-alkylscopolaminium salt and nitroglycerin, which has been cooled to room temperature, to the desired form for administering the medicament.

5. Process according to claim 4, characterised in that, in step 1a), instead of water an isotonic solution is used as the solvent.

6. Process according to claim 4, characterised in that in step 1b) ethanol, polyethylene glycol, propylene glycol or glycerin is used as the organic solvent.

7. Process according to one of claims 4 to 6, characterised in that the further processing in step 2) is carried out by

a) sterile filtering, packing and sterilising the solution,

b) mixing the solution with suitable adjuvants, packing it into a spray bottle or a spray can and, optionally, providing it with a propellant, or

c) reacting the solution with suitable adjuvants and processing it further to give a medicament in the form of soft gelatine capsules, rectal capsules, ointments, plasters, dragees, tablets or pills.

8. Use of N-alkylscopolaminium salt with 1 to 10 carbon atoms in the alkyl residue, preferably n-butylscopolaminium bromide, together with nitroglycerin for preparing a medicament according to one of claims 1 to 3.

**Claims for the Contracting States ES and GR**

1. Process for preparing a medicament which contains as active ingredient N-alkylscopolaminium salt with 1 to 10 carbon atoms in the alkyl residue, preferably N-butylscopolaminium bromide and, as further active ingredient, nitroglycerin as well as, optionally, conventional adjuvants and carriers, characterised in that

1a) an aqueous solution of N-alkylscopolaminium salt, preferably N-butylscopolaminium bromide, and nitroglycerin is prepared by introducing first approximately 90% of the total intended amount of water, acidifying it to a pH value of 3.0 to 6.5, heating it to 90-95°C, carefully introducing, whilst stirring, nitroglycerin as such, as a solution or deposited on an inert carrier material, adding N-alkylscopolaminium salt to the solution after the nitroglycerin has completely dissolved and, when the N-alkylscopolaminium salt has been dissolved, cooling the solution to room temperature and making it up with water to the desired final volume, or

1b) preparing an organic solution of N-alkylscopolaminium salt, preferably N-butylscopolaminium bromide, by introducing first the N-alkylscopolaminium salt and reacting it with 1 to 200 times the amount of an organic solvent with gentle heating in a water bath and adding a solution of nitroglycerin in alcohol or medium-chain-length triglycerides to the resulting solution, which has been cooled down to room temperature, and, optionally

2) processing in a manner known per se the obtained solution of N-alkylscopolaminium salt and nitroglycerin, which has been cooled to room temperature, to the desired form for administering the medicament.

2. Process according to claim 1, characterised in that, in step 1a), instead of water an isotonic solution is used as the solvent.

3. Process according to claim 1, characterised in that in step 1b) ethanol, polyethylene glycol, propylene glycol or glycerin is used as the organic solvent.

4. Process according to one of claims 1 to 3, characterised in that the further processing in step 2) is carried out by

a) sterile filtering, packing and sterilising the sol-

5

ution,

b) reacting the solution with suitable adjuvants, packing it into a spray bottle or a spray can and, optionally, providing it with a propellant, or

c) reacting the solution with suitable adjuvants and processing it further to give a medicament in the form of soft gelatine capsules, rectal capsules, ointments, plasters, dragees, tablets or pills.

5. Use of N-alkylscopolaminium salt with 1 to 10 carbon atoms in the alkyl residue, preferably n-butylscopolaminium bromide, together with nitroglycerin for preparing a medicament for treating colic pains in the region of the discharging urinary passages, the gall bladder and the bile duct.

## Revendications

### Revendications pour les Etats contractants BE, DE, FR, IT, LU, NL, AT, SE, CH (LI), GB

1. Médicament qui contient comme principe actif un sel de N-Alkylscopolamine comportant 1 a 10 atomes de carbone dans le radical alkyle, de préférence le bromure de N-butylscopolamine, ainsi que, le cas échéant, des adjuvants et véhicules usuels, caractérisé en ce qu'il contient de la nitroglycérine comme principe actif supplémentaire.

2. Médicament selon la revendication 1, caractérisé en ce qu'il contient un sel de N-alkylscopolamine et de la nitroglycérine dans un rapport pondéral compris entre 50 : 1 et 2 : 1 et de préférence entre 10: 1 et 7 : 1.

3. Médicament selon la revendication 1 ou 2, caractérisé en ce qu'une dose unitaire contient 10 à 30 mg et de préférence 18 à 22 mg de sel de N-alkylscopolamine et 0,4 à 4 mg et de préférence 1 a 3 mg de nitroglycérine.

4. Procédé pour préparer un medicament selon l'une quelconque des revendications 1 a 3, caractérisé en ce que

1a) on prépare une solution aqueuse de sel de N-alkylscopolamine comportant 1 à 10 atomes de carbone dans le radical alkyle, de préférence le bromure de M-butylscopolamine, et de nitroglycérine, en apportant environ 90% de la quantité totale d'eau prévue, en acidifiant a un pH de 3,0 a 6,5, en chauffant entre 90 et 95°C, en ajoutant avec précaution, sous agitation, la nitroglycérine telle quelle, sous forme de solution ou supportée par un matériau véhicule inerte, en ajoutant à la solution le sel de N-alkylscopolamine après dissolution complète de la nitroglycérine et en refroidissant à la température ambiante après dissolution ultérieure du sel de N-alkylscopolamine, et on complète avec de l'eau jusqu'au volume final souhaité, ou

1b) on prépare une solution ou suspension organique de sel de M-alkylscopolamine, de préférence le bromure de N-butylscopolamine, en apportant le sel de N-alkylscopolamine et en le mélangeant, au bain-marie sous chauffage modéré, avec une quantité 1 à 200 fois supérieure d'un solvant organique, et on ajoute à la solution résultante, refroidie à la température ambiante, une solution de nitroglycérine dans l'alcool ou dans des triglycérides à chaîne moyenne et le cas échéant,

2) on convertit ultérieurement la solution de sel de N-alkylscopolamine et de nitroglycérine obtenue, refroidie à la température ambiante, à la forme de présentation souhaitée pour le médicament, par des moyens connus par eux-mêmes.

5. Procédé selon la revendication 4, caractérisé en ce qu'on utilise dans l'étape 1a) une solution isotonique comme solvant à la place de l'eau.

6. Procédé selon la revendication 4, caractérisé en ce qu'on utilise dans l'étape 1b) l'éthanol, le polyéthylèneglycol, le propylèneglycol ou la glycérine comme solvant organique.

7. Procédé selon l'une quelconque des revendications 4 à 6, caractérisé en ce qu'on effectue la transformation ultérieure dans l'étape 2 en

a) filtrant en aseptie, en transvasant et en stérilisant la solution,

b) en mélangeant la solution avec des adjuvants appropriés, en en remplissant une bombe ou un flacon atomiseur et, le cas échéant, en lui adjoignant un agent propulseur ou

c) en mélangeant la solution avec des adjuvants appropriés et en la convertissant en un medicament sous forme de gélules, suppositoires, pommades, emplâtres, dragées, tablettes ou pilules.

8. Utilisation d'un sel de N-alkylscopolamine comportnnt 1 à 10 atomes de carbone dans le radical alkyle, de préférence le bromure de N-butylscopolamine, conjointement avec la nitroglycérine pour la fabrication d'un medicament conformément à l'une quelconque des revendications 1 à 3.

### Revendications pour les Etats contractants ES et GR

1. Procédé pour préparer un médicament qui contient comme principe actif un sel de N-alkylscopolamine comportant 1 à 10 atomes de carbone dans le radical alkyle, de préférence le bromure de N-butylscopolamine, et comme principe actif supplémentaire de la nitroglycérine ainsi que, le cas échéant, des adjuvants et véhicules usuels, caractérisé en ce que

1a) on prépare une solution aqueuse de sel de N-alkylscopolamine, de préférence le bromure de N-butylscopolamine, et de nitroglycérine, en apportant environ 90% de la quantité totale d'eau prévue, en acidifiant a un pH de 3,0 a 6,5, en

chauffant entire 90 et 95°C, en ajoutant avec précaution, sous agitation, la nitroglycérine telle quelle, sous forme de solution ou supportée par un matériau véhicule inerte, en ajoutant à la solution le sel de N-alkylscopolamine apses dissolution complète de la nitroglycérine et en refroidissant à la température ambiante après dissolution ultérieure du sel de N-alkylscopolamine, et on complète avec de l'eau jusqu'au volume final souhaité, ou

1b) on prépare une solution ou suspension organique de sel de N-alkylscopolamine, de préférence le bromure de N-butylscopolamine, en apportant le sel de N-alkylscopolamine et en le mélangeant, au bain-marie sous chauffage modéré, avec une quantité 1 à 200 fois supérieure d'un solvant organique, et on ajoute à la solution résultante, refroidie à la température ambiante, une solution de nitroglycérine dans l'alcool ou dans des triglycérides à chaîne moyenne, et le cas échéant,

2) on convertit ultérieurement la solution de sel de N-alkylscopolamine et de nitroglycérine obtenue, refroidie à la température ambiante, à la forme de présentation souhaitée pour le médicament, par des moyens connus par eux-mêmes.

2. Procédé selon la revendication 1, caractérisé en ce qu'on utilise dans l'étape 1a) une solution isotonique comme solvant à la place de l'eau.

3. Procédé selon la revendication 1, caractérisé en ce qu'on utilise dans l'étape 1b) l'éthanol, le polyéthylèneglycol, le propylèneglycol ou la glycérine comme solvant organique.

4. Procédé selon l'une quelconque des revendications 1 à 3, caractérisé en ce qu'on effectue la transformation ultérieure dans l'étape 2 en

a) filtrant en aseptie, en transvasant et en stérilisant la solution,

b) en mélangeant la solution avec des adjuvants appropriés, et en remplissant une bombe ou un flacon atomiseur et, le cas échéant, en lui adjoignant un agent propulseur ou

c) en mélangeant la solution avec des adjuvants appropriés et en la convertissant en un medicament sous forme de gélules, suppositoires, pommades, emplâtres, dragées, tablettes ou pilules.

5. Utilisation d'un sel de N-alkylscopolamine comportant 1 à 10 atomes de carbone dans le radical alkyle, de préférence le bromure de N-butylscopolamine, conjointement avec la nitroglycérine pour la fabrication d'un médicament pour le traitement des douleurs spasmodiques, principalement dans le domaine des voies d'élimination urinaires, de la vésicule biliaire et des canaux biliaires.